# EUROPEAN PATENT SPECIFICATION

(11) **EP 0 941 092 B1**
(45) Date of publication and mention of the grant of the patent: **29.12.2004**
(21) Application number: 97952019.4
(22) Date of filing: 25.11.1997
(51) Int. Cl.: A61K 31/535, A61K 31/445

(54) **USE OF NK-1 RECEPTOR ANTAGONISTS FOR TREATING MAJOR DEPRESSIVE DISORDERS**
VERFAHREN UND VORRICHTUNG ZUR KOMPENSATION VON DURCH CHROMINANZSIGNALVERARBEITUNG VERURSACHTEN LUMINANZSTÖRUNGEN
UTILISATION D'ANTAGONISTES DU RECEPTEUR NK-1 DANS LE TRAITEMENT DES ETATS DEPRESSIFS MAJEURS

(30) Priority: 02.12.1996 GB 9625051; 24.01.1997 GB 9701459; 27.06.1997 GB 9713715; 04.08.1997 GB 9716485; 07.10.1997 GB 9721190
(43) Date of publication of application: 15.09.1999
(73) Proprietor: MERCK SHARP & DOHME LTD., Hoddesdon Hertfordshire EN11 9NU (GB)
(72) Inventor: BAKER, Raymond, North Star Avenue Swindon SN2 1UH (GB); CURTIS, Neil, Roy, Harlow Essex CM20 3QR (GB); ELLIOTT, Jason, Matthew, Harlow Essex CM20 2QR (GB); HARRISON, Timothy, Harlow Essex CM20 2QR (GB); HOLLINGWORTH, Gregory, John, Harlow Essex CM20 2QR (GB); JACKSON, Philip, Stephen, Harlow Essex CM20 2QR (GB); KULAGOWSKI, Janusz, Jozef, Harlow Essex CM20 2QR (GB); RUPNIAK, Nadia, Melanie, Harlow Essex CM20 2QR (GB); SEWARD, Eileen, Mary, Harlow Essex CM20 2QR (GB); SWAIN, Christopher, John, Harlow Essex CM20 2QR (GB); WILLIAMS, Brian, John, Harlow Essex CM20 2QR (GB)
(74) Representative: Horgan, James Michael Frederic
(86) International application number: PCT/EP1997/006682
(87) International publication number: WO 1998/024438

(56) References cited:
- EP-A- 0 577 394
- WO-A-95/08549
- WO-A-95/18124
- WO-A-96/05181
- US-A- 5 496 833

## Description

This invention relates to the treatment or prevention of certain depressive disorders by the administration of a specific class of NK-1 receptor antagonists.

A major depressive episode has been defined as being a period of at least two weeks during which, for most of the day and nearly every day, there is either depressed mood or the loss of interest or pleasure in all, or nearly all activities. The individual may also experience changes in appetite or weight, sleep and psychomotor activity; descreased energy; feelings of worthlessness or guilt; difficulty thinking, concentrating or making decisions; and recurrent thoughts of death or suicidal ideation, plans or attempts. One or more major depressive episodes may give rise to a diagnosis of major depressive disorder (Diagnostic and Statistical Manual of Mental Disorders, Fourth Edition, American Psychiatric Association, 1994) .

Treatment regimens commonly include the use of tricyclic antidepressants (TCAs), monoamine oxidase inhibitors (MAOIs), some psychotropic drugs, lithium carbonate, and electroconvulsive therapy (ECT) (see R. J. Baldessarini in *Goodman & Gilman's The Pharmacological Basis of Therapeutics,* 9th Edition, Chapter 19, McGraw-Hill, 1996 for a review). More recently, new classes of antidepressant drugs are being developed including selective serotonin reuptake inhibitors (SSRIs), specific monoamine reuptake inhibitors and 5-HT_{1A} receptor agonists and antagonists.

The most established drug treatment for the management of depressive illness are the tricyclic antidepressants. For instance, depressed patients with prominent sleep disturbance and anxiety may be treated with a sedating tricyclic antidepressant such as amitriptyline; for other patients, less sedating compounds such as imipramine or desipramine can be used. As well as inhibiting the uptake of noradrenaline and 5-hydroxytriptamine, tricyclic antidepressants also possess antagonist properties at a variety of neurotransmitter receptors, including muscarinic cholinergic receptors, α₁-adrenoceptors and H₁-histamine receptors. These receptor antagonist effects account for much of the side-effect profile of the tricyclic antidepressants, and in particular, their anticholinergic side-effects which are particularly troublesome in patients with prostatic enlargement or glaucoma. Other side-effects include dry mouth, tachycardia, difficulty in visual accommodation, constipation, urinary retention, sexual dysfunction, cognitive impairment, postural hypotension, and weight gain.

Monoamine oxidase inhibitors are generally prescribed for patients who have failed to respond to tricyclic antidepressants or electroconvulsive therapy. As with tricyclic antidepressants, there are a number of side-effects associated with the use of MAOIs including dizziness, muscular twitching, insomnia, confusion, mania, tachycardia, postural hypotension, hypertension, dry mouth, blurred vision, impotence, peripheral oedema, hepatocellular damage and leucopenia.

Of the new classes of antidepressant, selective serotonin reuptake inhibitors are increasingly prescribed, particularly in patients where the use of tricyclic antidepressants is contraindicated because of their anticholinergic and cardiotoxic effects. SSRIs such as fluoxetine, fluvoxamine, sertraline and paroxetine are generally non-sedating. Furthermore, SSRIs do not stimulate appetite and may therefore be appropriate in patients in whom weight gain would be undesirable. However, SSRIs are not without their own side-effects, including nausea, diarrhoea, dry mouth, reduced appetite, dyspepsia, vomiting, headache, nervousness, insomnia, anxiety, tremour, dizziness, fatigue, decreased libido, pharyngitis, dyspnoea, skin rash and sexual dysfunction.

Whatever drug is used, there is a delay of usually two, three or even four weeks before a therapeutic effect is observed. This period of delay may be particularly difficult for a patient suffering from a major depressive illness.

Neurokinin 1 (NK-1; substance P) receptor antagonists are being developed for the treatment of a number of physiological disorders associated with an excess or imbalance of tachykinins, and in particular substance P. Examples of conditions in which substance P has been implicated include disorders of the central nervous system such as anxiety, depression and psychosis (see, for instance, International (PCT) patent specification Nos. WO 95/16679, WO 95/18124 and WO 95/23798).

On the other hand, European Patent Specification No. 0 286 928 describes inhibitors of the enzyme prolyl-endopeptidase, which enzyme degrades neuropeptides such as substance P, the enzyme inhibitors having an antipsychotic, anxiolytic and antidepressant action. Thus, degrading substance P or reducing the action of substance P in some other way (e.g. antagonism at its preferred NK-1 receptor) might be expected to be detrimental to the treatment of depression.

More recently, International (PCT) patent specification No. WO 96/24353 (published 15th August 1996) suggests that a more efficacious and safe treatment of psychiatric disorders would be achieved using a combination of a tachykinin antagonist and a serotonin agonist or selective serotonin reuptake inhibitor (SSRI). However, such as regimen would not be free of side-effects due to the serotonin agonist or SSRI.

NK-1 receptor antagonists are described in published European Patent Specification Nos. 0 360 390, 0 394 989, 0 429 366, 0 443 132, 0 482 539, 0 512 901, 0 512 902, 0 514 273, 0 514 275, 0 517 589, 0 520 555, 0 522 808, 0 528 495, 0 532 456, 0 533 280, 0 536 817, 0 545 478, 0 577 394, 0 590 152, 0 599 538, 0 610 793, 0 634 402, 0 686 629, 0 693 489, 0 694 535, 0 699 655, 0 699 674, 0 707 006, 0 708 101, 0 714 891, 0 723 959, 0 733 632 and 0 776 893; and in International Patent Specification Nos. 90/05525, 90/05729, 91/09844, 91/18899, 92/01688, 92/06079, 92/12151, 92/15585, 92/17449, 92/20661, 92/20676, 92/21677, 93/00330, 93/00331, 93/01159, 93/01165, 93/01169, 93/01170, 93/06099, 93/09116, 93/10073, 93/14113, 93/18023, 93/19064, 93/21155, 9321181, 93/23380, 93/24465, 94/01402, 94/02461, 94/03429, 94/03445, 94/04494, 94/04496, 94/05625, 94/07843, 94/10165, 94/10167, 94/10168, 94/10170, 94/11368, 94/13639, 94/13663, 94/14767, 94/15903, 94/19320, 94/19323, 94/20500, 94/26735, 94/26740, 94/29309, 95/02595, 95/04040, 95/04042, 95/06645, 95/07886, 95/07908, 95/08549, 95/11880, 95/14017, 95/15311, 95/16679, 95/17382, 95/18124, 95/18129, 95/19344, 95/20575, 95/21819, 96/22525, 95/23798, 95/26338, 95/28418, 95/30674, 95/30687, 96/05193, 96/05203, 96/06094, 96/07649, 96/1C562, 96/16939, 96/18643, 96/20197, 96/21661, 96/29304, 96/29317, 96/29326, 96/29328, 96/31214, 96/32385, 96/37489, 97/01553, 97/01554, 97/03066, 97/08144, 97/14671, 97/17362, 97/18206, 97/19084, 97/19942 and 97/21702; and in British Patent Specification Nos. 2 266 529, 2 268 931, 2 269 170, 2 269 590, 2 271 774, 2 292 144, 2 293 168, 2 293 169, and 2 302 689.

In view of the short-comings of existing antidepressant therapy, there is a need for new, safe and effective treatment for major depressive disorders.

The present invention provides the use of a CNS penetrant NK-1 receptor antagonist in an oral, once-a-day medicament far the treatment of major depressive disorder. The compounds of this class advantageously exhibit a rapid onset of action and a reduced side-effect profile when compared against conventional antidepressant agents.

In particular, the present invention provides a means for the identification of NK-1 receptor antagonists which would be effective in an oral once-a-day medicament for the treatment of major depressive disorders. The aforementioned patent specifications which describe NK-1 receptor antagonists provide no reliable method for the identification of such compounds.

The exceptional pharmacology of the class of NK-1 receptor antagonists of use in the present invention enables the treatment of major depressive disorders, without the need for concomitant therapy using tricyclic antidepressants or monoamine oxidase inhibitors or, in particular, without the need for concomitant use of a serotonin agonist or antagonist or an SSRI.

Furthermore, the exceptional pharmacology of the class of NK-1 receptor antagonists of use in the present invention results in a rapid onset of action.

The present invention accordingly provides the use of an orally active, long acting, CNS-penetrant NK-1 receptor antagonist (as hereinafter defined) for the manufacture of a medicament adapted for oral administration for the treatment or prevention of major depressive disorder without concomitant therapy with other antidepressant agents.

There exists a patient population in whom major depressive disorder is inadequately treated with existing antidepressant therapy. Furthermore, some patients may be adversely affected by the side-effects of tricyclic antidepressant drugs.

The present invention accordingly provides the use of an orally active, long acting, CNS-penetrant NK-1 receptor antagonist for the manufacture of a medicament adapted for oral administration for the treatment or prevention of major depressive disorder without concomitant therapy with other antidepressant agents in a patient who is non-responsive to heterocyclic antidepressants (TCAs, tetracyclics, and the like), SSRIs, mixed serotonin and norepinephrine selective reuptake inhibitors, dopamine reuptake inhibitors or MAOIs, or for whom heterocyclic antidepressants (TCAs, tetracyclics, and the like), SSRIs, mixed serotonin and norepinephrine selective reuptake inhibitors, dopamine reuptake inhibitors or MAOIs are contraindicated.

In particular, there exists a patient population in whom major depressive disorder is inadequately treated with SSRIs or serotonin agonists or antagonists. Furthermore, some patients may be adversely affected by the side-effects of SSRIs or serotonin agonists or antagonists.

The present invention accordingly provides the use of an orally active, long acting, CNS-penetrant NK-1 receptor antagonist for the manufacture of a medicament adapted for oral administration for the treatment or prevention of major depressive disorder without concomitant therapy with other antidepressant agents in a patient who is non-responsive to SSRIs or serotonin agonists or antagonists or for whom SSRIs or serotonin agonists or antagonists are contraindicated.

As used herein, the term "non-responsive" in relation to major depressive disorder means patients who have not had a reasonable clinical response (e.g. a 50% reduction in Hamilton Depression Scale (HAM-D) from a patient's baseline score after treatment with one or more clinical courses of conventional antidepressants).

As used herein, the term "major depressive disorder" includes single or recurrent major depressive episodes, with or without psychotic features, catatonic features, melancholic features, atypical features or postpartum onset and, in the case of recurrent episodes, with or without interepisode recovery and with or without seasonal pattern.

Other mood disorders encompassed within the term "major depressive disorder" include dysthymic disorder with early or late onset and with or without atypical features; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

Major depressive disorders may also result from a general medical condition including, but not limited to, myocardial infarction, diabetes, miscarriage or abortion, etc.

A "major depressive episode" is defined as at least two weeks of depressed mood or loss of interest, which may be accompanied by other symptoms of depression. The symptoms must persist for most of the day (i.e. for at least two thirds of the patients' waking hours), nearly every day (i.e. for at least ten out of fourteen days) for at least two consecutive weeks. A "depressed mood" is often described by the patient as feeling sad, hopeless, helpless or worthless. The patient may also appear sad to an observer, for example, through facial expression, posture, voice and tearfulness. In children and adolescents, the mood may be irritable. A "loss of interest" is often described by the patient as feeling less interested in hobbies or not feeling any enjoyment in activities that were previously considered to be pleasurable.

A major depressive episode may be accompanied by other symptoms of depression including significant weight loss when not dieting or weight gain (e.g. a change of more than 5% body weight in one month), or decrease or increase in appetite; insomnia or hypersomnia; psychomotor agitation or retardation; fatigue or loss of energy; feelings of worthlessness or excessive or inappropriate guilt; diminished ability to think or concentrate; or indecisiveness; and recurrent thoughts of death, recurrent suicidal ideation with or without a specific plan, or a suicide attempt.

As used herein, the term "treatment" refers both to the treatment and to the prevention or prophylactic therapy of the aforementioned conditions.

Preferred NK-1 receptor antagonists for use in the present invention are selected from the classes of compounds described in European Patent Specification No. 0 577 394, and International Patent Specification Nos. 95/08549, 95/18124, 95/23798 and 96/05181, and International Patent Application No. PCT/GB97/01630. The preparation of such compounds is fully described in the aforementioned publications.

Particularly preferred NK-1 receptor antagonists of use in the present invention include:
2-(S)-(3,5-bis(trifluoromethyl)benzyloxy)-3(S)-(4-fluorophenyl)-4-(3-(5-oxo-1H,4H-1,2,4-triazolo)methyl)morpholine;
2-(R)-(1-(R)-(3,5-bis(trifluoromethyl)phenyl)ethoxy)-4-(3-(5-oxo-1H,4H-1,2,4-triazolo)methyl)-3-(S)-phenyl-morpholine;
2-(S)-(3,5-bis(trifluoromethyl)benzyloxy)-4-(3-(5-oxo-1H,4H-1,2,4-triazolo)methyl)-3-(S)-phenyl-morpholine;
2-(R)-(1-(R)-(3,5-bis(trifluoromethyl)phenyl)ethoxy)-3-(S)-(4-fluorophenyl)-4-(3-(5-oxo-1H,4H-1,2,4-triazolo)methyl)morpholine;
2-(R)-(1-(R)-(3,5-bis(trifluoromethyl)phenyl)ethoxy)-4-(5-(N,N-dimethylamino)methyl-1,2,3-triazol-4-yl)methyl-3-(S)-phenylmorpholine;
2-(R)-(1-(R)-(3,5-bis(trifluoromethyl)phenyl)ethoxy)-4-(5-(N,N-dimethylamino)methyl-1,2,3-triazol-4-yl)methyl-3-(S)-(4-fluorophenyl)morpholine;
2-(R)-(1-(R)-(3,5-bis(trifluoromethyl)phenyl)ethoxy)-3-(S)-(4-fluorophenyl)-4-(3-(4-monophosphoryl-5-oxo-1H-1,2,4-triazolo)methyl)morpholine;
2-(R)-(1-(R)-(3,5-bis(trifluoromethyl)phenyl)ethoxy)-3.(S).(4-nuorophenyl)-4-(3-(1-monophosphoryl-5-oxo-1H-1,2,4-triazolo)methyl)morpholine;
2-(R)-(1-(R)-(3,5-bis(triffuoromethyl)phenyl)ethoxy)-3-(S)-(4-fluorophenyl)-4-(3-(2-monophosphoryl-5-oxo-1H-1,2,4-triazolo)methyl)morpholine;
2-(R)-(1-(R)-(3,5-bis(trifluoromethyl)phenyl)ethoxy)-3-(S)-(4-fluorophenyl)-4-(3-(5-oxyphosphoryl-1H-1,2,4-triazolo)methyl)morpholine;
2-(S)-(1-(R)-(3,5-bis(trifluoromethyl)phenyl)ethoxy)-3-(S)-(4-fluorophenyl)-4-(3-(1-monophosphoryl-5-oxo-4H-1,2,4-triazolo)methyl)morpholine;
2-(R)-(1-(R)-(3,5-bis(trifluoromethyl)phenyl)ethoxy)-4-(4-N,N-dimethylaminobut-2-yn-yl)-3-(S)-(4-fluorophenyl)morpholine;
(3S,5R,6S)-3-[2-cyclopropoxy-5-(trifluoroxnethoxy)phenyl]-6-phenyl-1-oxa-7-aza-spiro[4.5]decane;
(3R,5R,6S)-3-[2-cyclopropoxy-5-(trifluoromethoxy)phenyl]-6-phenyl-1-oxa-7-aza-spiro[4.5]decane;
or a pharmaceutically acceptable salt thereof.

Full descriptions of the preparation of the NK-1 receptor antagonists which may be employed in the present invention may be found in the references cited herein.

Suitable pharmaceutically acceptable salts of the NK-1 receptor antagonists of use in the present invention include acid addition salts which may, for example, be formed by mixing a solution of the compound with a solution of a pharmaceutically acceptable non-toxic acid such as hydrochloric acid, fumaric acid, maleic acid, succinic acid acetic acid, citric acid, tartaric acid, carbonic acid, phosphoric acid or sulphuric acid, Salts of amine groups may also comprise the quaternary ammonium salts in which the amino nitrogen atom carries an alkyl, alkenyl, alkynyl or aralkyl group. Where the compound carries an acidic group, for example a carboxylic acid group, the present invention also contemplates salts thereof, preferably non-toxic pharmaceutically acceptable salts thereof, such as the sodium, potassium and calcium salts thereof.

Preferably the compositions containing an NK-1 receptor antagonist of use according to the present invention are in unit dosage forms such as tablets, pills, capsules, wafers and the like. Additionally, the NK-1 receptor antagonists of use according to the present invention may be presented as granules or powders for extemporaneous formulation as volume defined solutions or suspensions. Alternatively, the NK-1 receptor antagonists of use according to the present invention may be presented in ready-prepared volume defined solutions or suspensions. Preferred forms are tablets and capsules.

For preparing solid compositions such as tablets, the principal active ingredient is mixed with a pharmaceutical carrier, e.g. conventional tableting ingredients such as corn starch, lactose, sucrose; sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

Compositions of the present invention may also be administered via the buccal cavity using conventional technology, for example, absorption wafers.

Compositions in the form of tablets, pills, capsules or wafers for oral administration are particularly preferred.

A minimum dosage level for the NK-1 receptor antagonist is about 1mg per day, preferably about 5mg per day and especially about 10mg per day. A maximum dosage level for the NK-1 receptor antagonist is about 1500mg per day, preferably about 1000mg per day and especially about 500mg per day. The compounds are administered once a day.

It will be appreciated that the amount of the NK-1 receptor antagonist required for use in the treatment or prevention of major depressive disorders will vary not only with the particular compounds or compositions selected but also with the route of administration, the nature of the condition being treated, and the age and condition of the patient, and will ultimately be at the discretion of the patient's physician or pharmacist.

Two compounds of use in the present invention which are described in International Patent Application No. PCT/GB97/01630 may be prepared according to the following methods:

### PREPARATION 1

### (2S)-1-tert-Butoxycarbonyl-2-phenylpiperidin-3-one

Dimethyl sulfoxide (20.80ml, 22.90g, 29.3mmol) in dichloromethane (75ml) was added dropwise to a cooled (-70°C) solution of oxalyl chloride (13.95ml, 20.30g, 160mmol) in dichloromethane (350ml). The mixture was stirred at -70°C for 15 minutes, then (2*S*,3*S*)-1-*tert*-butoxycarbonyl-3-hydroxy-2-phenylpiperidine (prepared by the method described in European Patent Specification number 0 528 495-A; 36.91g, 133mmol) in dichloromethane (150ml) was added dropwise. The mixture was stirred at -70 °C for 20 minutes, then allowed to warm to -30°C. The mixture was cooled to -50 °C and triethylamine (55.95ml, 40.45g, 400mmol) was added slowly. The mixture was allowed to warm to 0°C and diluted with ice-cooled dichloromethane (250ml). The mixture was washed with ice cold aqueous citric acid solution (5%, 2x300ml) and water (300ml), dried (MgSO₄), and the solvent was evaporated under reduced pressure to give the title compound as a yellow oil (42.3g), which was used immediately without further purification. ¹H NMR (250MHz, CDCl₃) δ 7.5-7.3 (5H, m), 5.8 (1H, br s), 4.2 (1H, br s), 3.4 (1H, m), 2.6 (2H, m), 2.0 (2H, m), and 1.54 (9H, s).

### PREPARATION 2

### (2S,3R)-1-tert-Butoxgcarbonyl-3-hydroxy-3-(2-methylene-3-phenoxypropyl)-2-phenylpiperidine

A solution of 3-(chloromagnesio)-2-(phenoxymethyl)-1-propene in THF (0.91M, 3ml) (Louw *et*. *al*., *Tetrahedron*, 48, 6087-6104, 1992, prepared from 2.74mmol of 3-chloro-2-(phenoxymethyl)-1-propene) was slowly added to a solution of (2*S*)-1-*tert*-butoxycarbonyl-2-phenylpiperidin-3-one (Preparation 1) in THF (3ml). The mixture was stirred at room temperature for 1 hours, then saturated aqueous ammonium chloride (20ml) was added and the mixture was extracted with ethyl acetate (20ml). The organic phase was washed with brine, dried (MgSO₄) and the solvent was evaporated under reduced pressure . The residue was purified by column chromatography on silica gel, eluting with hexane/ethyl acetate (100:0 increasing to 80:20) to give the title compound. ¹H NMR (360MHz, CDCl₃) δ 7.48 (2H, d, *J*=6.9 Hz), 7.35-7.2 (6H, m), 6.9-6.88.(3H, m), 5.4 (1H, s), 5.15 (2H, d, *J*=13.7 Hz), 4.61 (2H, s), 4.11 (2H, m), 3.17 (1H, m), 2.66 and 2.59 (2H, AB d, *J*=14.0 Hz), 1.95 (2H, m), 1.79 (2H, m), and 1.36 (9H, s). m/z (ES⁺) 424 (M+1).

### PREPARATION 3

### (5R,6S)-3-Methylene-6-phenyl-1-oxa-7-(tert-butoxycarbonyl)aza-spiro[4.5]decane

To a cooled(-80 °C) solution of (2*S*,3*R*)-1-*tert*-butoxycarbonyl-3-hydroxy-3-(2-methylene-3-phenoxypropyl)-2-phenylpiperidine (Preparation 2, 1.53g, 3.62mmol) in THF (20ml) was added n-butyl lithium (2.5M in hexanes, 1.45ml, 3.62mmol) followed by a solution of zinc chloride (0.5M in THF, 7.24ml, 3.62mmol). The solution was allowed to warm to room temperature and tetrakis(triphenylphosphine)palladium (0) (0.23g, 0.2mmol) was added. The mixture was degassed with bubbling nitrogen and heated under reflux for 16 hours. The mixture was cooled and the solvent was evaporated under reduced pressure. The residue was partitioned between ethyl acetate and 2M sodium hydroxide. The organic phase was washed with saturated brine, dried (MgSO₄) and purified by chromatography on a column containing silica gel (eluting with hexane containing increasing proportions of ethyl acetate between 0% to 5%). Evaporation of the fractions gave *(6S,5R)-3-methylene-6-phenyl-1-oxa-7-(tert-butoxycarbonyl)aza-spiro[4.5]decane*. ¹H NMR (360MHz, CDCl₃) δ 7.58 (2H, d, *J*=8.4 Hz), 7.32-7.21 (3H, m), 5.23 (1H, s), 5.06 (1H, m), 4.97 (1H, m), 4.39 (2H, AB d, *J*=13.3 Hz), 3.99 (1H, dd, *J*=13.3, 4.48 Hz), 2. 83 (1H, ABd *J*=15.5 Hz), 2.7 (1H,td *J*=12.5, 3.93 Hz), 2.5 (1H, ABd, *J*=15.4 Hz), 2.15 (2H, td, *J*=12., .4 Hz), 1.69 (2H, m), and 1.46 (9H,s). m/z (ES⁺) 329 (M+2H-^{t}BuOCO).

### PREPARATION 4

### (5R,6S)-3-Keto-6-phenyl-1-oxa-7-(tert-butoxycarbonyl)aza-spiro[4.5]decane

Through a cooled (-80 °C) solution of (5*R*,6*S*)-3-methylene-6-phenyl-1-oxa-7-(*tert*-butoxycarbonyl)aza-spiro[4.5]decane (Preparation 3; 0.665g) in dichloromethane (5ml) and methanol (5ml) was bubbled a mixture of ozone and oxygen for 45 minutes. After the solution had been purged with nitrogen, dimethyl sulphide (0.5ml) was added and then stirred under nitrogen at room temperature for 16 hours. The solvent was removed *in vacuo* and the residue partitioned between ethyl acetate and water. The organic phase was dried (MgSO₄), evaporated and the residue purified by chromatography on a column containing silica gel (eluting with hexane containing increasing proportions of ethyl acetate between 0% to 10%). Evaporation of the fractions gave the title compound. ¹H NMR (250MHz, CDCl₃) δ 7.58 (2H, d, *J*=6.2 Hz), 7.37-7.26 (3H, m), 5.3 (1H, s), 4.15 and 4.09 (2H, AB d, *J*=17.4 Hz), 3.97 (1H, m), 2.80 (1H, td, *J*=12.9, 4.0 Hz), 2.74 and 2.48 (2H, ABd, *J*=18.1 Hz), 2.29 (2H, m), 1.88-1.63 (2H, m), and 1.44 (9H, s). m/z (ES⁺) 332 (M+1).

### PREPARATION 5

### (5R,6S)-3-Trifluoromethylsulfonyloxy-6-phenyl-1-oxa-7-(tert-butoxycarbonyl)aza-spiro[4.5]dec-3-ene

To a cooled (-80 °C) solution of 1M sodium hexamethyldisilazide (0.38ml, 0.38mmol) in THF was added a solution of (5*R*,6*S*)-3-keto-6-phenyl-1-oxa-7-(*tert*-butoxycarbonyl)aza-spiro[4.5]decane (Preparation 4; 0.105mg, 0.319mmol) in THF (3ml). The solution was stirred for I hours at -80°C then a solution of 2-[N,N-bis(trifluoromethylsulfonyl)amino]-5-chloropyridine (0.163g, 0.415mmol) in THF (3ml) was added. The solution was stirred at -80°C for 30 minutes then at room temperature for 30 minutes before being quenched by addition of saturated ammonium chloride solution and ethyl acetate. The dried (MgSO₄) organic phase was purified by chromatography on a column containing silica gel (eluting with hexane containing increasing proportions of ethyl acetate between 0% to 5%). Evaporation of the fractions gave the title compound. ¹H NMR (360MHz, CDCl₃) δ 7.4 (2H, d, *J*=7.3 Hz), 7.3-7.22 (3H, m), 6.01 (1H, t, *J*=2.13 Hz), 5.13 (1H, s), 4.56 and 4.26 (2H, ABdd, *J*=12.4, 1.97 Hz),4.10 (1H, dt, *J*=12.6, 4.22 Hz), 3.00 (1H, m), 2.28-2.04 (2H, m), 1.88-1.76 (2H, m), and 1.37 (9H, s). m/z (ES⁺) 464 (M+1).

### PREPARATION 6

### (5R,6S)-3-Trimethylstannyl-6-phenyl-1-oxa-7-(tert-butoxycarbonyl)aza- spiro[4.5]dec-3-ene

To a degassed solution of (5*R*,6*S*)-3-trifluoromethylsulfonyloxy-6-phenyl-1-oxa-7-(*tert*-butoxycarbonyl)aza-spiro[4.5]dec-3-ene (Preparation 5; 0.482g, 1.04mmol), lithium chloride (0.264g, 6.25mmol), lithium carbonate (0.076g) and hexamethyl distannane(0.96g, 2.9mmol) in THF (10ml) was added triphenylphosphine palladium (0) (0.06g). The solution was degassed and then heated at 60°C for 5 hours under nitrogen. Water (20ml) and ethyl acetate (20ml) were added and the dried organic phase was purified by chromatography on a column containing silica gel (eluting with hexane containing increasing proportions of ethyl acetate between 0% to 5%). Evaporation of the fractions gave the title compound as a crystalline solid. ¹H NMR (360MHz, CDCl₃) δ 7.25 (2H, d, *J*=7.3 Hz), 7.1-7.0 (3H, m), 5.83 (1H, t, *J*=2.5 Hz), 4.78 (1H, s), 4.48 and4.02 (2H, dd, *J*=12.9, 2.3 Hz), 3.96 (1H, dd, *J*=6.16, 13.4 Hz), 2.95 (1H, td, *J*=13.3, 4.5 Hz), 1.84 (1H, m), 1.68 (1H, m), 1.60 (2H, m), 1.19 (9H, s), and 0.0 (6H, s).

### PREPARATION 7

### (2S,3R)-1-tert-Butoxycarbonyl-3-(3-hydroxypropyn-1-yl)-2-phenylpiperidin-3-ol

O-Trimethylsilylpropargyl alcohol (24.51ml, 20.47g, 160ml) was added slowly to a cooled (-10°C) solution of ethylmagnesium bromide (1M in tetrahydrofuran, 160ml, 160mmol). The mixture was stirred at 0°C for 20 minutes, then at room temperature for 2 hours. The mixture was cooled to -10°C and a solution of (2*S*)-1-*tert*-butoxycarbonyl-2-phenylpiperidin-3-one (Preparation 1; 42.3g) in tetrahydrofuran (200ml) was added dropwise over 30 minutes. (Internal temperature below -5°C). The mixture was stirred at room temperature for 14 hours, poured into water (300ml) and saturated aqueous ammonium chloride (300ml) and extracted with ethyl acetate (2x300ml). The combined organic fractions were washed with brine (300ml), dried (MgSO₄) and the solvent was evaporated under reduced pressure. The residue was dissolved in ethyl acetate (500ml) and a solution of tetrabutylammonium fluoride (1M in THF, 160ml, 160mmol) was added dropwise. The mixture was stirred at room temperature for 30 minutes, water (300ml) was added, and the layers were separated. The aqueous layer was extracted with ethyl acetate (2x300ml) and the combined organic fractions were washed with water (300ml) and brine (300ml), dried (MgSO₄) and the solvent was evaporated under reduced pressure to give the crude title compound as an orange oil (45g). The crude material was purified by flash column chromatography on silica gel, eluting with hexane/ethyl acetate (90:10 increasing to 25:75) to give the title compound as an amber oil (32.2g). ¹H NMR (CDCl₃) δ 7.53-7.55 (2H, m), 7.19-7.35 (3H, m), 5.56 (1H, s), 4.27 (2H, s), 3.99-4.03 (1H, m), 3.25 (1H, br s), 2.77-2.81 (1H, m), 2.77 (1H, br s), 2.12-2.20 (1H, m), 1.91-1.99 (2H, m), 1.77-1.83 (1H, m), and 1.39 (9H, s).

### PREPARATION 8

### 2-Bromo-4-(trifluoromethoxy)phenol

To a cooled (0 °C) solution of 4-trifluoromethoxyphenol (35.6g, 0.2mol) in chloroform (280ml) was added dropwise a solution of bromine (32g, 0.2mol) in chloroform (50ml). The solution was stirred at 0°C for 1 hour and at room temperature for 2 hours. Dichloromethane (200ml) and water (400ml) ware added and the organic phase was washed further with water(400ml), brine (200ml) and dried (MgSO₄). The solvent was removed and the residue was purified by distillation at reduced pressure to give the title compound. ¹H NMR (250MHz, CDCl₃) δ 7.38 (1H, d, *J*=2.1 Hz), 7.13 (1H, dd, *J*=9.1, 2.1 Hz), 7.03 (1H, d, *J*=9.1 Hz), and 5.53 (1H, s).

### PREPARATION 9

### 2-Benzyloxy-5-(trifluoromethoxy)bromobenzene

2-Bromo-4-(trifluoromethoxy)phenol (Preparation 8; 5g, 20mmol) was dissolved in *N*,*N*-dimethylformamide (60ml), and potassium carbonate (5.4g, 40mmol) was added, followed by benzyl bromide (3.5ml, 30mmol), and the reaction was stirred at ambient temperature for 15 hours. The reaction was diluted with water (150ml) and extracted into ethyl acetate (3x60ml). The combined organic fractions were washed with water (100ml), brine (100ml), dried (MgSO₄) and evaporated *in vacuo*. Purification on silica, eluting with 2% and 5% ethyl acetate in hexane gave the title compound as a clear oil (6.7g, 96%). ¹H NMR (250MHz, CDCl₃) δ 5.47 (2H, s), 7.23 (1H, d, *J*=9 Hz), 7.43 (1H, dd *J*=8.2, 2.9 Hz), and 7.75 (6H, m).

### PREPARATION 10

### Z-(2S,3R)-1-tert-Butoxycarbonyl-3-(3-hydroxyprop-1-en-1-yl)-2-phenylpiperidin-3-ol

Palladium on calcium carbonate, poisoned with lead (Lindlar catalyst, 2g) was added to a solution of (2*S*,3*R*)-1-*tert*-butoxycarbonyl-3-(3-hydroxypropyn-1yl)-2-phenylpiperidin-3-ol (Preparation 7; 32g, 96.6mmol) in ethyl acetate (300ml) and the mixture was stirred under hydrogen (1 atmosphere) for 4 hours. The mixture was filtered and the solvent was evaporated under reduced pressure to give the title compound as an oil (32g, 100%). ¹H NMR (360MHz, CDCl₃) δ 7.42 (2H, d, *J*=7.6 Hz), 7.35-7.25 (3H, m), 5.83 (1H, d, *J*12.3 Hz), 5.68 (1H, dt, *J*=12.3, 6.0 Hz), 5.06 (1H, s), 4.27. (1H, m), 4.12 (2H, m), 3.32 (1H, m), 3.13 (1H, s), 2.28 (1H, t, *J*=5.9 Hz), 2.02 (1H, m), 1.92-1.78 (3H, m), and 1.32 (9H, s). m/z (ES⁺) 334 (M+1).

### PREPARATION 11

### (5R,6S)-6-Phenyl-1-oxa-7-(tert-butoxycarboyl)aza-spiro[4.5]dec-3-ene

Diethylazodicarboxylate (18.2ml, 115mmol) in THF (100ml) was added dropwise to a solution of *Z*-(2*S*,3*R*)-1-*tert*-butoxycarbonyl-3-(3-hydroxyprop-1-en-1-yl)-2-phenylpiperidin-3-ol (Preparation 10; 32g, 96mmol) and triphenylphosphine (30.2g, 115mmol) in THF (700ml). The mixture was stirred at 0°C for 30 minutes then at room temperature for 1.5 hours. The solvent was evaporated under reduced pressure and the residue was purified by flash column chromatography on silica gel, eluting with hexane/ethyl acetate (95:5 increasing to 80:20) to give the title compound as a colorless solid (23.4g, 77%). ¹H NMR (CDCl₃) δ 7.45 (2H, d, *J*=7.4 Hz), 7.27 (2H, t, *J*=7.4 Hz), 7.20 (1H, t, *J*=7.4 Hz), 6.03 (1H, dt, *J*=6.1, 2.0 Hz), 5.68 (1H, dt, *J*=6.1, 2.0 Hz), 5.06 (1H, s), 4.61 (1H, dt, *J*=13.1, 2.0 Hz), 4.32 (1H, dt, *J*=13.1, 2.0 Hz), 4.08 (1H, m). 3.05 (1H, m), 2.05 (1H, m), 1.75 (3H, m), and 1.37 (9H, s). m/z (ES⁺) 316 (M+1).

### PREPARATION 12

### 2-Benzyloxy-5-(trifluoromethoxy)benzene

Benzyl bromide (66.17ml, 95.35g, 0.56mol) was added to a mixture of 4-(trifluoromethoxy)phenol (90.26g, 0.51mo1) and potassium carbonate (140.97g, 1.2mol) in dimethylformamide (160ml) and the mixture was stirred at room temperature for 72 hours. The mixture was poured into water (1.51) and extracted with ethyl acetate (3x500ml). The combined organic fractions were washed with aqueous sodium carbonate (saturated, 500ml), dried (MgSO₄) and the solvent was evaporated under reduced pressure to give the title compound as a colorless solid (133.5g, 99%). ¹H NMR (360MHz, CDCl₃) δ 7.39 (5H, m), 7.14 (2H, d, *J*=9.0 Hz), 6.95 (2H, d, *J*=9.0 Hz), and 5.05 (2H, s).

### PREPARATION 13

### 2-Benzyloxy-5-(trifluoromethoxy)iodobenzene

Iodine (71.96g, 0.28mol) in chloroform was added dropwise to a mixture of 2-benzyloxy-5-(trifluoromethoxy)benzene (Preparation 12, 73.06g, 0.27mol) and silver trifluoroacetate (71.57g, 0.32mol) in dichloromethane and the mixture was stirred at room temperature for 18 hours. The mixture was filtered through celite, washed with aqueous sodium thiosulfate (5%, 2x2 1), dried (MgSO₄) and the solvent was evaporated under reduced pressure. The residue was purified by flash column chromatography on silica gel, eluting with hexane/ethyl acetate, to give the title compound as a colorless oil (108.03g), containing 11% unreacted 2-benzyloxy-5-(trifluoromethoxy)iodobenzene. ¹H NMR (360MHz, CDCl₃) δ 7.67 (1H, d, *J*=2.8 Hz), 7.40 (5H, m), 7.16 (1H, dd, *J*=8.9, 2.8 Hz), 6.82 (1H, d, *J*=8.9 Hz), and 5.14 (2H, s).

### PREPARATION 14

### (5R,6S)-3-(2-Benzyloxy-5-(trifluoromethoxy)phenyl)-6-phenyl-1-oxa-7-(tert-butoxycarbonyl)aza-spiro[4.5]dec-3-ene

(5*R*,6*S*)-3-Trimethylstannyl-6-phenyl-1-oxa-7-(*tert*-butoxycaxbonyl)aza-spiro[4.5]dec-3-ene (Preparation 6; 6.43mmol), lithium chloride (0.163g), benzyloxy-5-(trifluoromethoxy)phenol (Preparation 9; 7.7mmol) in toluene (25ml) was degassed before addition of triphenylphosphine palladium (0) (0.37g). The solution was degassed thoroughly before heating to 110°C for 14 hours. The solution was partitioned between water and ethyl acetate and the dried organic phase was purified by chromatography on a column containing silica gel (eluting with hexane containing increasing proportions of ethyl acetate between 0% to 4%) to give the title compound. ¹H NMR (360MHz, CDCl₃) δ 1.33 (9H, s), 1.65 (1H, m), 1.76 (2H, m), 2.08 (1H, m), 3.11 (1H, m), 4.08 (1H, m), 4.60 (1H, dd, *J*=12.2 Hz, *J*=2 Hz), 4.92 (1H, dd, *J*=12.1 Hz, *J*=1.8 Hz), 5.08 (1H, s), 5.1 (2H, q, *J*=11.5 Hz), 6.65 (1H, s), 6.94 (2H, d, *J*=8.9 Hz), 7.08 (1H, d, *J*=9 Hz), 7.18 (2H, t, *J*=8.1 Hz), 7.25 (3H, m), 7.38 (5H, m).

### PREPARATION 15

### (3S,5R,6S)-3-(2-Hydroxy-5-(trifluoromethoxy)phenyl)-6-phenyl-1-oxa-7-(tert-butoxycarbonyl)aza-spiro[4.5]decane

(5*R*,6*S*)-3-(2-Benzyloxy-5-(trifluoromethoxy)phenyl)-6-phenyl-1-oxa-7-(*tert*-butoxycarbonyl)aza-spiro[4.5]dec-3-ene (Preparation 14) (3.88g) was dissolved in ethyl acetate (15ml) and methanol (15ml). Palladium hydroxide on carbon (1.00g) was added and the suspension was shaken under a hydrogen atmosphere (50 psi) for 72 hours. The mixture was filtered and the solvent was evaporated under reduced pressure. The residue was purified by medium pressure chromatography on silica gel, eluting with hexane/ethyl acetate (75:25) to give *(3R*,*5R*,*εS)-3-(2-hydroxy-5-(trifluoromethoxy)phenyl)-6-phenyl-1-oxa-7-(tert-butoxycarbonyl)aza-spiro[4.5]decane* (191mg), ¹H NMR (250MHz, CDCl₃) δ 7.70 (2H, d, *J*=7.3 Hz), 7.33 (2H, t, *J*=7.3 Hz), 7.26 (1H, d, *J*=7.3 Hz), 7.05 (1H, br s), 6.96 (2H, m), 6.82 (1H, d, *J*=9.4 Hz), 5.43 (1H, s), 4.27 (1H, m), 4.01 (1H, m), 3.95 (1H, m), 3.73 (1H, m), 2.73 (2H, m), 2.33 (1H, m), 1.87-1.58 (4H, m); and 1.50 (9H, s).and *(3S,5R,6S)-3-(2-hydroxy-5-(trifluoromethoxy)phenyl)-6-phenyl-1-oxa-7-(tert-butoxycarbonyl)aza-spiro[4.5]decane* (2.3g), ¹H NMR (360MHz, CDCl₃) δ 1.38 (9H, s), 1.73 (2H, m), 1.81 (1H, m), 2.18 (2H, m), 2.50 (1H, m), 2.81 (1H, m), 3.62 (1H, t, *J*=7.2 Hz), 3.92 (1H, m), 3.98 (1H, d, *J*=13.2 Hz), 4.23 (1H, m), 5.33 (1H, s), 6.75 (1H, d, *J*=8.5 Hz), 6.94 (2H, m), 7.25 (1H, m), 7.31 (2H, m), and 7.55 (2H, d, *J*=7.8 Hz).

### PREPARATION 16

### (3R,5R,6S)-3-(2-Benzyloxy-5-(trifluoromethoxy)phenyl)-6-phenyl-1-oxa-7-(tert-butoxycarbonyl)aza-spiro[4.5]decane

A mixture of 2-benzyloxy-5-(trifluoromethoxy)iodobenzene (Preparation 13, 21.8g, 55.2mmol), (5*R*,6*S*)-6-phenyl-1-oxa-7-(*tert*-butoxycarbonyl)aza-spiro[4.5]dec-3-ene (Preparation 11, 7.0g, 22.1mmol), tetra-n-butylammonium chloride (6.18g, 22.2mmol), lithium chloride (9.35g, 0.22mol) and potassium formate (5.64g, 67.0mmol) in dimethylformamide (100ml) was degassed with a firestone valve (5 x). Palladium acetate (491mg, 2.2mmol) was added and the mixture was degassed with a firestone valve (5 x). The mixture was stirred at 60°C for 15 hours, then further 2-benzyloxy-5-(trifluoromethoxy)iodobenzene (Preparation 13, 4.32g, 11.0mmol), potassium formate (2.78g, 33.5mmol) and palladium acetate (260mg, 1.1mmol) were added. The mixture was stirred at 60°C for 22 hours, cooled and filtered. The solvent was evaporated under reduced pressure, water (600ml) was added and the mixture was extracted with ethyl acetate (2x300ml). The combined organic fractions were washed with brine (300ml), dried (MgSO₄) and the solvent was evaporated under reduced pressure. The residue was purified by flash column chromatography on silica gel, eluting with hexane/dichloromethane (75:25 increasing to 0:100) then dichloromethane/ethyl acetate (95:5), to give the title compound (9.42g, 73%). ¹H NMR (360MHz, CDCl₃) δ 7.56 (2H, d, *J*=7.7 Hz), 7.40-7.20 (8H, m), 7.14 (1H, d, *J*=2.0 Hz), 7.00 (1H, dd, *J*=8.9, 2.0 Hz), 6.88 (1H, d, *J*=8.9 Hz), 5.30 (1H, s), 5.08 (2H, s), 4.27 (1H, m), 3.97 (1H, m), 3.87 (2H, m), 2.78 (1H, m), 2.56 (1H, m), 2.15 (1H, m), 1.96 (1H, m), 1.67 (3H, m), and 1.42 (9H, s).

### PREPARATION 17

### (3R,5R,6S)-3-(2-Hydroxy-5-(trifluoromethoxy)phenyl)-6-phenyl-1-oxa-7-(tert-butoxycarbonyl)aza-spiro[4.5]decane

Palladium on carbon (10%, 0.59g) was added to a solution of (3*R*,5*R*,6*S*)-3-(2-benzyloxy-5-(trifluoromethoxy)phenyl)-6-phenyl-1-oxa-7-(*tert*-butoxycarbonyl)aza-spiro[4.5]decane (Preparation 16, 6.10g, 10.5mmol) in methanol-water (99:1, 200ml) and the mixture was stirred under hydrogen (50 psi.) for 72 hours. The mixture was filtered, washing with ethanol, and the solvent was evaporated under reduced pressure. The residue was purified by flash column chromatography on silica gel, eluting with dichloromethane/ethyl acetate (99:1 increasing to 90:10) to give the title compound. ¹H NMR (360MHz, CDCl₃) δ 7.70 (2H, d, *J*=7.3 Hz), 7.33 (2H, t, *J*=7.3 Hz), 7.26 (1H, d, *J*=7.3 Hz), 7.05 (1H, br s), 6.96 (2H, m), 6.82 (1H, d, *J*=9.4 Hz), 5.43 (1H, s), 4.27 (1H, m). 4.01 (1H, m), 3.95 (1H, m), 3.73 (1H, m), 2.73 (2H, m), 2.33 (1H, m), 1.87-1.58 (4H, m), and 1.50 (9H, s).

### PREPARATION 18

### (3S,5R,6S)-3-[2-(1-Phenylthiocycloprop-1-yl)oxy-5-(trifluoromethoxy)phenyl]-6-phenyl-1-oxa-7-(tert-butoxycarbonyl)aza-spiro[4.5]decane

(3*S*,5*R*,6*S*)-3-(2-Hydmxy-5-(trifluoromethoxy)phenyl)-6-phenyl-1-oxa-7-(*tert*-butoxycarbonyl)aza-spiro[4.5]decane (Preparation 15) (290mg, 0.59mmol) was dissolved in toluene (5ml) and silver carbonate (179mg, 0.65mmol) was added in one portion. (1-Iodocycloprop-1-yl)phenylsulfide (Cohen T. and Matz J. R., *J. Am. Chem. Soc.* **1980,** *102,* 6902) (180mg, 0.65mmol) was then added over one minute at room temperature. The mixture was stirred at 55°C for 4 hours, then further portions of silver carbonate (179mg, 0.65mmol) and (1-iodocycloprop-1-yl)phenylsulfide (180mg, 0.65mmol) were added. The mixture was stirred at 55°C for a further 3 hours, cooled, filtered and the solvent was evaporated under reduced pressure. The residue was purified by column chromatography on silica gel, eluting with hexane/ethyl acetate (90: 10 increasing to 80:20) to give the title compound as a colourless oil (120mg, 32%). ¹H NMR (250MHz, CDCl₃) δ 7.55-7.44 (4H, m), 7.36-7.23 (7H, m), 7.13-7.02 (2H, m), 5.16 (1H, br s), 4.09 (1H, t, *J*=6 Hz), 4.03-3.92 (1H, m), 3.67-3.49 (2H, m), 2.94-2.79 (1H, m), 2.26 (1H, dd, *J*=7.9, 12.9 Hz), 2.15-2.01 (2H, m), 1.76-1.59 (3H, m), 1.53-1.45 (4H, m), and 1.36 (9H, s). m/z (ES⁺) 642 (M+1).

### PREPARATION 19

### (3R,5R,6S)-3-[2-(1-Phenylthiocycloprop-1-yl)oxy-5-(trifluoromethoxy)phenyl]-6-phenyl-1-oxa-7-(tert-butoxycarbonyl)aza-spiro[4.5]decane

Prepared from (3*R*,5*R*,6*S*)-3-(2-hydroxy-5-(trifluoromethoxy)phenyl)-6-phenyl-1-oxa-7-(*tert*-butoxycarbonyl)aza-spiro[4.5]decane (Preparation 17) according to the method of Preparation 18. ¹H NMR (360MHz, CDCl₃) δ 7.57 (2H, app. d, *J*=7.6 Hz), 7.45 (2H, app. d, *J*=7.7 Hz), 7.36-7.19 (7H, m), 7.16-7.06 (2H, m), 5.28 (1H, br s), 4.13 (1H, app. t, *J*=7.8 Hz), 3.96 (1H, br. d, *J*=13 Hz), 3.80-3.60 (2H, m), 2.79 (1H, br. t, *J*=13 Hz), 2.50 (1H, dd, *J*=13, 7.9 Hz), 2.17 (1H, dt, *J*=13, 4.6 Hz), 1.80 (1H, dd, J=12, 9.8 Hz), 1.75-1.38 (7H, m), and 1.44 (9H, s). m/z (ES⁺) 642 (M+1).

### PREPARATION 20

### (3S,5R,6S)-3-[2-Cyclopropoxy-5-(trifluoromethoxy)phenyl]-6-phenyl-1-oxa-7-(tert-butoxycarbonyl)aza-spiro[4.5]decane

Naphthalene (120mg, 0.936mmol) was dissolved in THF (1.5ml) under nitrogen and freshly cut lithium metal (7.0mg, 0.94mmol) was added. The mixture was then sonicated at room temperature for 20 minutes to produce a dark green solution of lithium naphthalenide. This solution was cooled to -78 °C, then (3*S*,5*R*,6*S*)-3-[2-(1-phenylthiocycloprop-1-yl)oxy-5-(trifluoromethoxy)phenyl]-6-phenyl-1-oxa-7-(*tert*-butoxycarbonyl)aza-spiro[4.5]decane (Preparation 18) (120mg, 0.187mmol) in THF (0.5ml) was added over 1 minute. The reaction mixture was stirred for 30 minutes, then water (5ml) and ether (10ml) were added. The layers were separated and the aqueous layer was extracted with ether (10ml). The combined organic fractions were dried (MgSO₄) and the solvent was evaporated under reduced pressure. The residue was purified by column chromatography on silica gel, eluting with hexane/ethyl acetate (90:10 increasing to 80:20) to give the title compound as a colourless oil (58.6mg, 59%). ¹H NMR (250MHz, CDCl₃) δ 7.58-7.52 (2H, m), 7.36-7.17 (4H, m), 7.10-7.01 (2H, m), 5.18 (1H, br s), 4.20 (1H, t, *J*=6.7 Hz), 4.05-3.95 (1H, m), 3.76-3.55 (3H, m), 2.92-2.79 (1H, m), 2.37 (1H, dd, *J*=12.9, 7.8 Hz), 2.18-2.06 (2H, m), 1.80-1.67 (3H, m), 1.38 (9H, s), and 0.86-0.73 (4H, m). m/z (ES⁺) 534 (M+1).

### PREPARATION 21

### (3R,5R,6S)-3-[2-Cyclopropoxy-5-(trifluoromethoxy)phenyl]-6-phenyl-1-oxa-7-(tert-butoxycarbonyl)aza-spiro[4.5]decane

Naphthalene (120mg, 0.936mmol) was dissolved in THF (1.5ml) under nitrogen and freshly cut lithium metal (7.0mg, 0.94mmol) was added. The mixture was then sonicated at room temperature for 20 minutes to produce a dark green solution of lithium naphthalenide. A solution of (3*R*,5*R*,6*S*)-3-[2-(1-phenylthiocycloprop-1-yl)oxy-5-(trifluoromethoxy)phenyl]-6-phenyl-1-oxa-7-(*tert*-butoxycarbonyl)aza-spiro[4.5]decane (Preparation 19, 135mg, 0.21mmol) in THF (2ml) under nitrogen was cooled to -78°C and the solution of lithium naphthalenide in THF was added dropwise until the intense green colour persisted. The reaction was then stirred for one minute, water (5ml) was added and the mixture was warmed to room temperature. Ether (10ml) was added and the layers were separated. The aqueous phase was extracted with a further portion of ether (10ml) and the combined organic phases were dried (MgSO₄) and the solvent was evaporated under reduced pressure. The residue was purified by column chromatography on silica gel, eluting with hexane/ethyl acetate (50:50) to give the title compound as a colourless oil (87mg, 78%). ¹H NMR (360MHz, CDCl₃) δ 7.59 (2H, app. d, *J*=7.6 Hz), 7.32 (2H, app. t, *J*=7.6 Hz), 7.27-7.18 (2H, m), 7.11-7.03 (2H, m), 5.32 (1H, br s), 4.29-4.21 (1H, m), 3.97 (1H, br. d, *J*=13 Hz), 3.83-3.68 (3H, m), 2.76 (1H, dt, *J*=13, 4.1 Hz), 2.55 (1H, dd, *J*=13, 7.2 Hz), 2.22 (1H, dt, *J*=12, 5.2 Hz), 1.85 (1H, dd, *J*=13, 9.9 Hz), 1.80-1.63 (3H, m), 1.46 (9H, s), and 0.82-0.76 (4H, m). m/z (ES⁺) 534 (M+1).

### COMPOUND A

### (3S,5R,6S)-3-[2-Cyclopropoxy-5-(trifluoromethoxy)phenyl]-6-phenyl-1-oxa-7-aza-spiro[4.5]decane Hydrochloride

Trifluoroacetic acid (2.5ml) was added dropwise to a stirred, cooled 0°C) solution of (3*S*,5*R*,6*S*)-3-[2-cyclopropoxy-5-(trifluoromethoxy)phenyl]-6-phenyl-1-oxa-7-(*tert*-butoxycarbonyl)aza-spiro[4.5]decane (Preparation 20; 492mg, 0.92mmol) in dichloromethane (25ml) and the mixture was stirred at room temperature for 3 hours. The mixture was poured into water (50ml), the pH was adjusted to 10.0 with aqueous sodium hydroxide (4M) and the mixture was extracted with dichloromethane (3x50ml). The combined organic fractions were dried (MgSO₄) and the solvent was evaporated under reduced pressure. The residue was purified by flash column chromatography on silica gel, eluting with dichloromethane/methanol/ammonia (aq.) (96:4:0.4 increasing to 94:6:0.6). The residue was dissolved in ethanol (20ml), cooled in ice and ethereal hydrogen chloride (1M, 1.8ml, 1.8mmol) was added dropwise. The mixture was stirred at 0°C for 5 minutes, then the solvent was evaporated under reduced pressure. The residue was crystallized from ether (20ml)/ethanol (0.5ml) and the solid was collected and dried *in vacuo* to give the title compound as a colorless solid (354mg, 89%). m.p. 214-216 °C, ¹H NMR (500MHz, CD₃OD) δ 7.59 (2H, m), 7.52 (3H, m), 7.26 (1H, d, *J*=8.9 Hz), 7.03 (1H, dd, *J*=8.9, 2.2 Hz), 6.20 (1H, d, *J*=2.2 Hz), 4.85 (2H, br s), 4.43 (1H, s), 4.19 (1H, t, *J*=8.0 Hz), 3.87 (3H, quin, *J*=8.0 Hz), 3.76 (1H, m), 3.44 (1H, m), 3.25 (2H, m) 2.29-1.78 (6H, m), 0.80 (2H, m), and 0.66 (2H, m). m/z (ES⁺) 434 (M+1). Found: C, 61.41; H, 5.51; N, 3.08. C₂₄H₂₆F₃NO₃.HCl requires: C, 61.34; H, 5.79; N, 2.98%.

### COMPOUND B

### (3R,5R,6S)-3-[2-Cyclopropoxy-5-(trifluoromethoxy)phenyl]-6-phenyl-1-oxa-7-aza-spiro[4.5]decane

Prepared from the compound of Preparation 21 according to the method used for Compound A. ¹H NMR (360MHz, CDCl₃) δ 7.60-7.42 (2H, m), 7.36-7.26 (3H, m), 7.03 (1H, d, *J*=8.9 Hz), 6.95 (1H, br. d, *J*=8.9 Hz), 6.81 (1H, br s), 3.92 (1H, t, *J*=7.4 Hz), 3.62-3.53 (2H, m), 3.50 (1H, s), 3.20 (1H, dd, *J*=12, 4.2 Hz), 2.77 (1H, dt, *J*=12, 2.8 Hz), 2.30-1.93 (4H, m), 1.87 (1H, br s), 1.71-1.49 (3H, m), 0.76-0.65 (2H, m), and 0.65-0.54 (2H, m). m/z (ES⁺) 434 (M+1).

A further compound and diastereomers thereof of use in the present invention may be prepared according to the following method:

### DESCRIPTION 1

### 2-(1-Phenylthiocycloprop-1-yl)oxy-5-(trifluoromethoxy)bemzaldehyde

Silver carbonate (1.2 g, 4.34 mmol) was added to a solution of 2-hydroxy-5-(trifluoromethoxy)benzaldehyde (0.5 g, 2.43 mmol) and (1-iodocycloprop-1-yl)phenylsulfide (Cohen T. and Matz J. R., *J. Am. Chem*. *Soc.* **1980**, *102*, 6902) (1.2 g, 4.34 mmol) in toluene (30 mL) and the mixture was stirred at 40 °C overnight. The mixture was cooled, diluted with ethyl acetate and filtered, washing well with ethyl acetate. The mixture was washed with aqueous sodium hydroxide, dried (MgSO₄) and the solvent was evaporated under reduced pressure. The residue was purified by flash column chromatography on silica gel, eluting with hexane/Et₂O (95:5), to give the title compound as a yellow oil (191 mg, 27%). ¹H NMR (360MHz, CDCl₃) δ 1.51-1.56 (2H, m), 1.44-1.48 (2H, m), 7.25-7.35 (7H, m), 7.69 (1H, d, *J* 2.0 Hz), and 10.26 (1H, s).

### DESCRIPTION 2

### 2-Cyclopropoxy-5-(trifluoromethoxy)benzaldehyde

Freshly cut lithium metal (97 mg, 13.9 mmol) was added to a solution of naphthalene (1.77 g, 13.9 mmol) in THF (20 mL) and the mixture was sonicated at room temperature for 30 min. to produce a dark green solution of lithium naphthalenide. A solution of 2-(1-phenylthiocycloprop-1-yl)oxy-5-(trifluoromethoxy)benzaldehyde (Description 1, 96 mg, 0.27 mmol) in THF (2 mL) was cooled to -78 °C and the solution of lithium naphthalenide in THF (2 mL) was added dropwise until the intense green colour persisted. The reaction was then stirred for 5 min., water (6 mL) was added and the mixture was warmed to room temperature. The mixture was extracted with ethyl acetate, the combined organic fractions were dried (MgSO₄) and the solvent was evaporated under reduced pressure. The residue was purified by flash column chromatography on silica gel, eluting with hexane/Et₂O (80:20), to give to give the title compound as a colourless oil (4 mg, 6%). ¹H NMR (360MHz, CDCl₃) δ 0.86 (4H, m), 3.82-3.9 (1H, m), 7.42 (2H, m), 7.62 (1H, d, *J* 2.5 Hz), and 10.36 (1H, s).

### DESCRIPTION 3

### 2-Nitro-4-(trifluoromethoxy)phenol

Iron(111)nitrate nonahydrate (1.97 g, 4.87 mmol) was added to a solution of 4-(trifluoromethoxy)phenol (2 g, 11.24 mmol) in ethanol (20 mL) and the mixture was heated under reflux overnight. The mixture was allowed to cool to room temperature, acidified to pH 1 with aqueous hydrochloric acid (1M) and extracted with ethyl acetate. The combined organic fractions were dried (MgSO₄), and the solvent was evaporated under reduced pressure. The residue was purified by short column chromatography on silica gel, eluting with hexane/EtOAc (70:30), to give the title compound as a yellow oil (2.25 g, 89%). ¹H NMR (360MHz, CDCl₃) δ 10.53 (1H, s), 8.01 (1H, d, *J* 3.0 Hz), 7.49 (1H, dd, *J* 9.1, 3.0 Hz), and 7.23 (1H, d, *J* 9.1 Hz).

### DESCRIPTION 4

### 2-(1-Phenylthiocycloprop-1-yl)oxy-5-(trifluoromethoxy)nitrobenzene

Prepared from the compound of Description 3 according to the method of Description 1. ¹H NMR (360MHz, CDCl₃) δ 7.73 (1H, d, *J* 2.7 Hz), 7.58 (1H, d, *J* 9.2 Hz), 7.50-7.24 (6H, m), 1.57-1.53 (2H, m), and 1.44-1.40 (2H, m).

### DESCRIPTION 5

### 2-Cyclonronoxy-5-(trifluoromethoxy)benzeneamine

Prepared from the compound of Description 4 according to the method of Description 2. ¹H NMR (360MHz, CDCl₃) δ 7.06 (1H, dd, *J* 2.8, 6.7 Hz), 6.56 (2H, m), 3.83 (2H, br s), 3.74 (1H, m), and 0. 79 (4H, m). m/z (ES⁺) 234 (M+1).

### DESCRIPTION 6

### 2-(1-Phenylthiocycloprop-1-yl)oxy-5-(trifluoromethoxy)benzeneamine

Iron powder (13.5 g, 241 mmol) was added to a suspension of 2-(1-phenylthiocycloprop-1-yl)oxy-5-(trifluoromethoxy)nitrobenzene (Description 4, 11.27 g, 30.1 mmol) in water (300 mL) and acetic acid (75 mL) and the mixture was stirred at 80 °C overnight. The mixture was cooled and filtered through celite, washing with ether. The filtrate was extracted with ether, the combined organic fractions were washed with aqueous sodium hydroxide (1M), dried (MgSO₄), and the solvent was evaporated under reduced pressure. The residue was purified by flash column chromatography on silica gel, eluting with hexane/Et₂O (90:10 increasing to 80:20), to give the title compound as a yellow solid (8 g, 78%). ¹H NMR (360MHz, CDCl₃) δ 7.48 (2H, m), 7.34-7.23 (3H, m), 7.15 (1H, d, *J* 8.74 Hz), 6.60-6.56 (2H, m), 3.78 (2H, br s), 1.49-1.46 (2H, m), and 1.39-1.35 (2H, m).

### DESCRIPTION 7

### 2-Cyclopropoxy-5-(trifluoromethoxy)benzeneamine

Prepared from the compound of Description 6 according to the method of Description 2. ¹H NMR (360MHz, CDCl₃) δ 7.06 (1H, dd, *J* 2.8, 6.7 Hz), 6.56 (2H, m), 3.83 (2H, br s), 3.74 (1H, m), and 0.79 (4H, m). m/z (ES⁺) 234 (M+1).

### DESCRIPTION 8

### 2-Cyclopropoxy-5-(trifluoromethoxy)iodobenzene

An ice-cooled solution of sodium nitrite (3.55 g, 51 mmol) in water (10 mL) was added dropwise to a stirred, cooled (0 °C) solution of 2-cyclopropoxy-5-(trifluoromethoxy)benzeneamine (Description 7, 4.8 g, 20.6 mmol) in aqueous hydrochloric acid (5M, 300 mL), maintaining the internal temperature at 0 °C. The mixture was stirred at 0 °C for 30 min., then potassium iodide (8.55 g, 51.5 mmol) in water (10 mL) was added dropwise, maintaining the internal temperature at 0 °C. The mixture was stirred at 0 °C for 30 min., then allowed to warm up to room temperature and stirred until nitrogen evolution ceased. The mixture was extracted with ether, the organic fraction was washed with aqueous sodium thiosulfate (10%), dried (MgSO₄), and the solvent was evaporated under reduced pressure. The residue was purified by flash column chromatography on silica gel, eluting with hexane/Et₂O (98:2 increasing to 95:5), to give the title compound as a colourless oil (6.23 g, 88%). ¹H NMR (360MHz, CDCl₃) δ 7.62 (1H, d, *J* 2.4 Hz), 7.20 (1H, dd, *J* 9.1, 2.4 Hz), 7.15 (1H, d, *J* 9.1 Hz), 3.80 (1H, m), and 0.83 (4H, m).

### DESCRIPTION 9

### 2-Cyclopropoxy-5-(trifluoromethoxy)benzaldehyde

A solution of 2-cyclopropoxy-5-(trifluoromethoxy)iodobenzene (Description 8, 0.344 g, 1 mmol) in toluene (2.5 mL) was degassed with bubbling nitrogen for 10 min. Tetrakis(triphenylphosphine)palladium (0) (15 mg) was added, the mixture was degassed with bubbling nitrogen for a further 5 min., then carbon monoxide was bubbled through the mixture for 10 min. The mixture was warmed to 50 °C and a solution of tributyl tin hydride (0.3 mL, 1.1 mmol) in toluene (5 mL) was added at a rate of 2 mL/h. *via* a syringe pump, maintaining carbon monoxide bubbling throughout. The mixture was cooled, diluted with ether (20 mL) and aqueous potassium fluoride solution (50%) was added. The mixture was stirred at room temperature overnight, filtered and the layers were separated. The organic layer was dried (MgSO₄), and the solvent was evaporated under reduced pressure. The residue was purified by flash column chromatography on silica gel, eluting with hexane/Et₂O (80:20), to give the title compound as a colourless oil. ¹H NMR (360MHz, CDCl₃) δ 0.86 (4H, m), 3.82-3.9 (1H, m), 7.42 (2H, m), 7.62 (1H, d, *J* 2.5 Hz), and 10.36 (1H, s).

### DESCRIPTION 10

### (±)-(2RS)-1-tert-Butoxycarbonyl-2-phenylpipepidin-3-one

Dimethyl sulfoxide (32.0 mL, 35.3 g, 0.45 mol) in dichloromethane (100 mL) was added dropwise to a cooled (-70 °C) solution of oxalyl chloride (18.7 mL, 27.5 g, 0.22 mol) in dichloromethane (1000 mL). The mixture was stirred at -70 °C for 15 min., then (2*S*,3*S*)-1-*tert*-butoxycarbonyl-3-hydroxy-2-phenylpiperidine (prepared by the method described in European Patent Specification number 0 528 495-A; 50 g, 0.18 mol) in dichloromethane (150 mL) was added dropwise. The mixture was stirred at -70 °C for 1 h., then triethylamine (125.8 mL, 91.3 g, 0.9 mol) was added slowly. The mixture was stirred at room temperature for 1 h., water (250 mL) and aqueous sodium hydrogen carbonate (saturated, 250 mL) were added and the mixture was stirred at room temperature overnight. The layers were separated and the aqueous layer was extracted with dichloromethane (2 x 300 mL). The combined organic fractions were washed with brine, dried (MgSO₄) and the solvent was evaporated under reduced pressure. The residue was purified by flash column chromatography on silica gel, eluting with hexane/EtOAc (90:10), to give the title compound as a yellow oil (45.0 g, 91%). ¹H NMR (250MHz, CDCl₃) δ 7.5-7.3 (5H, m), 5.8 (1H, br s), 4.2 (1H, br s), 3.4 (1H, m), 2.6 (2H, m), 2.0 (2H, m), and 1.54 (9H, s).

### DESCRIPTION 11

### (±)-(2R3R,2S3S)-1-(tert-Butoxycarbonyl)-2-phenylpiperidin-3-amine

A solution of hydroxylamine hydrochloride (17 g, 0.24 mol) and sodium acetate (55.67 g, 0.41 mol) in water (150 mL) was added to a solution of (±)-(2*RS*)-1-*tert*-butoxycarbonyl-2-phenylpiperidin-3-one (Description 10, 45 g, 0.16 mol) in ethanol (300 mL) and the mixture was stirred at room temperature for 1 h. The solvent was evaporated under reduced pressure, water was added and the mixture was extracted with ethyl acetate. The organic fraction was washed with brine, dried (MgSO₄) and the solvent was evaporated under reduced pressure- The residue was dissolved in ethanol (400 mL) and Raney nickel (50 g) was added. The mixture was shaken under hydrogen (40 psi) overnight, filtered and the solvent was evaporated under reduced pressure. The residue was purified by flash column chromatography on silica gel, eluting with CH₂Cl₂/MeOH (100:0 increasing to 85:15), to give the title compound as a colorless oil (10.9 g, 24%). ¹H NMR (360MHz, CDCl₃) δ 7.43 (2H, d, *J* 7.0 Hz), 7.30 (3H, m), 5.19 (1H, d, J 6.2 Hz), 4.00 (1H, m), 3.17 (2H, m), 1.90-1.64 (4H, m), 1.36 (9H, s), and 1.26 (2H, br s).

### COMPOUND C

### (±)-(2R3R,2S3S)-N-{[2-Cyclopropoxy-5-(trifluoromethoxy)phenyl]methyl}-2-phenylpiperidin-3-amine Dihydrochloride

2-Cyclopropoxy-5-(trifluoromethoxy)benzaldehyde (Description 9, 55 mg, 0.21 mmol) was added to (±)-(2*R*3*R*,2*S*3*S*)-1-(*tert*-butoxycarbonyl)-2-phenylpiperidin-3-amine (Description 11, 58 mg, 0.21 mmol), citric acid (89 mg, 0.42 mmol) and 3Å molecular sieves in dry methanol (5 mL) and the mixture was stirred at room temperature for 1.5 h. Sodium borohydride (30 mg) was added and the mixture was stirred at room temperature for 2 h. Ethyl acetate was added and the mixture was washed with aqueous hydrochloric acid (0.1M, 2 x 25 mL) and brine (25 mL), dried (MgSO₄) and the solvent was evaporated under reduced pressure. The residue was dissolved in dichloromethane (3 mL), cooled to 0 °C and trifluoroacetic acid (2 mL) was added slowly. The mixture was stirred at room temperature for 1 h., the solvent was evaporated under reduced pressure and ethyl acetate was added. The mixture was washed with aqueous sodium hydrogen carbonate (saturated, 2 x 25 mL) and brine (25 mL), dried (MgSO₄) and the solvent was evaporated under reduced pressure. The residue was purified by flash column chromatography on silica gel, eluting with CH₂Cl₂/MeOH/NH₃(Aq.) (96:4:0.4). The residue was dissolved in ethanol (2 mL), cooled in ice and ethereal hydrogen chloride (1M, 0.24 mL, 0.24 mmol) was added. The solvent was evaporated under reduced pressure and the residue was recrystallised from ethanol to give the title compound as a colorless solid (20 mg, 20%). m.p. 169-171 °C. ¹H NMR (400MHz, CD₃OD) δ 0.64 (1H, m), 0.80 (3H, m), 1.99 (1H, m), 2.24 (1H, m), 2.46 (2H, m), 3.30 (1H, m), 3.64 (1H, m), 3.75 (2H, m), 3.96 (1H, br s), 4.08 (1H, m), 4.95 (1H. s). 7.23 (1H, s), 7.31 (1H, d, *J* 9.0 Hz), 7.37 (1H, d, *J* 9.0 Hz), 7.54 (3H, m), and 7.67 (2H, m). m/z (ES⁺) 407 (M+1).

Particularly preferred NK-1 receptor antagonists of use in the present invention are compounds which are potent NK-1 receptor antagonists, i.e. compounds with an NK-1 receptor affinity (IC₅₀) of less than 10nM, favourably less than 2nM and preferably less than 1nM.

The class of orally active, long acting, CNS-penetrant NK-1 receptor antagonists of use in the present invention is identified using a combination of the following assays:

### ASSAY 1: NK-1 Receutor binding

NK-1 receptor binding assays are performed in intact Chinese hamster ovary (CHO) cells expressing the human NK-1 receptor using a modification of the assay conditions described by Cascieri *et al*, *J. Pharmacol*. *Exp. Ther*., 1992, **42**, 458. Typically, the receptor is expressed at a level of 3x10⁵ receptors per cell. Cells are grown in monolayer culture, detached from the plate with enzyme-free dissociation solution (Speciality Media Inc.), and washed prior to use in the assay. ¹²⁵I-Tyr⁸-substance P (0.1nM, 2000Ci/mmol; New England Nuclear) is incubated in the presence or absence of test compounds (dissolved in 5µl dimethylsulphoxide, DMSO) with 5x10⁴ CHO cells. Ligand binding is performed in 0.25ml of 50mM Tris-HCl, pH7.5, containing 5mM MnCl₂, 150mM NaCl, 0.02% bovine serum albumin (Sigma), 50µg/ml chymostatin (Peninsula), 0.1nM phenylmethylsulphonyl fluoride, 2µg/ml pepstatin, 2µg/ml leupeptin and 2.8µg/ml furoyl saccharine. The incubation proceeds at room temperature until equilibrium is achieved (>40 minutes) and the receptor-ligand complex is harvested by filtration over GF/C filters pre-soaked in 0.1% polyethylenimine using a Tomtek 96-well harvester. Nonspecific binding is determined using excess substance P (1µM) and represents <10% of total binding.

### ASSAY 2: Gerbil Foot-Tapping

CNS-penetrant NK-1 receptor antagonists for use in the present invention can be identified by their ability to inhibit foot tapping in gerbils induced by anxiogenic agents (such as pentagastrin) or central infusion of NK-1 receptor agonists such as GR73632, or caused by aversive stimulation such as foot shock or single housing, based on the method of Rupniak & Williams, *Eur*. *J. Pharmacol*., 1994, **265,** 179.

Male or female Mongolian gerbils (35-70g) are anaesthetised by inhalation of an isoflurane/oxygen mixture to permit exposure of the jugular vein in order to permit administration of test compounds or vehicle in an injection volume of 5ml/kg i.v. Alternatively, test compounds may be administered orally or by subcutaneous or intraperitoneal routes. A skin incision is then made in the midline of the scalp to expose the skull. An anxiogenic agent (e.g. pentagastrin) or a selective NK-1 receptor agonist (e.g. GR73632 (d Ala[L-Pro⁹,Me-Leu¹⁰]-substance P-(7-11)) is infused directly into the cerebral ventricles (e.g. 3pmol in 5µl i.c.v., depending on test substance) by vertical insertion of a cuffed 27 gauge needle to a depth of 4.5mm below bregma. The scalp incision is closed and the animal allowed to recover from anaesthesia in a clear perspex observation box (25cm x 20cm x 20cm). The duration and/or intensity of hind foot tapping is then recorded continuously for approximately 5 minutes. Alternatively, the ability of test compounds to inhibit foot tapping evoked by aversive stimulation, such as foot shock or single housing, may be studied using a similar method of quantification.

### ASSAY 3: Ferret Emesis

Individually housed male ferrets (1.0 -2.5 kg) are dosed orally by gavage with test compound. Ten minutes later they are fed with approximately 100g of tinned cat food. At 60 minutes following oral dosing, cisplatin (10mg/kg) is given i.v. *via* a jugular vein catheter inserted under a brief period of halothane anaesthesia. The catheter is then removed, the jugular vein ligated and the skin incision closed. The ferrets recover rapidly from the anaesthetic and are mobile within 10-20 minutes. The animals are observed continuously during recovery from the anaesthetic and for 4 hours following the cisplatin injection, after which time the animals are killed humanely. The numbers of retches and vomits occurring during the 4 hours after cisplatin administration are recorded by trained observers.

### ASSAY 4: Separation-Induced Vocalisation

Male and female guinea-pigs pups are housed in family groups with their mothers and littermates throughout the study. Experiments are commenced after weaning when the pups are 2 weeks old. Before entering an experiment, the pups are screened to ensure that a vigorous vocalisation response is reproducibly elicited following maternal separation. The pups are placed individually in an observation cage (55cm x 39cm x 19cm) in a room physically isolated from the home cage for 15 minutes and the duration of vocalisation during this baseline period is recorded. Only animals which vocalise for longer than 5 minutes are employed for drug challenge studies (approximately 50% of available pups may fail to reach this criterion). On test days each pup receives an oral dose or an s.c. or i.p. injection of test compound or vehicle and is then immediately returned to the home cage with its mother and siblings for 30 to 60 minutes (or for up to 4 hours following an oral dose, dependent upon the oral pharmacokinetics of the test compound) before social isolation for 15 minutes as described above. The duration of vocalisation on drug treatment days is expressed as a percentage of the pre-treatment baseline value for each animal. The same subjects are retested once weekly for up to 6 weeks. Between 6 and 8 animals receive each test compound at each dose tested.

As used herein, the term "CNS-penetrant" refers to NK-1 receptor antagonists which are able to inhibit NK-1 receptor agonist-induced foot-tapping in the gerbil as hereinafter defined.

Essentially, hind foot-tapping in the gerbil induced by infusion of the NK-1 receptor agonist, GR73632 (d Ala[L-Pro⁹,Me-Leu¹⁰]-substance P-(7-11)), under anaesthesia, directly into the central ventricles is inhibited when a CNS-penetrant NK-1 receptor antagonist is administered intravenously immediately prior to GR73632 challenge, wherein hind foot-tapping over a period of five minutes following recovery from the anaesthesia is inhibited with an ID₅₀≤3mg/kg, and preferably with an ID₅₀≤1mg/kg.

In an alternative method, the NK-1 receptor antagonist is administered orally, 1 hour prior to GR73632 challenge, wherein the foot-tapping over a period of five minutes following recovery from anaesthesia is inhibited with an ID₅₀≤30mg/kg, and preferably with an ID₅₀≤10mg/kg.

CNS-penetrant NK-1 receptor antagonists of use in the present ivnention are also effective in the attenuation of separation-induced vocalisations by guinea-pig pups as hereinafter defined.

Essentially, a vocalisation response in guinea-pig pups is induced by isolation from their mothers and littermates, which response is attenuated when a CNS-penetrant NK-1 receptor antagonist is administered subcutaneously 30 minutes prior to isolation, wherein vocalisations during the first 15 minutes of isolation are attenuated with an ID₅₀≤20mg/kg, preferably with an ID₅₀≤10mg/kg, and especially with an ID₅₀≤5mg/kg.

In an alternative method, the NK-1 receptor antagonist is administered orally, 4 hours prior to isolation, wherein vocalisations during the first 15 minutes of isolation are attenuated with an ID₅₀≤20mg/kg, preferably with an ID₅₀≤10mg/kg, and especially with an ID₅₀≤5mg/kg.

A suitable selection cascade for NK₁ antagonists of use according to the present invention is as follows:
(i) Determine affinity for human NK₁ receptor in radioligand binding studies (Assay 1); select compounds with IC₅₀ ≤ 10nM, preferably IC₅₀ ≤ 2nM, especially IC₅₀ ≤ 1nM.
(ii) Determine ability of compounds to penetrate CNS by their ability to inhibit foot tapping in gerbils induced by central injection of an NK₁ agonist (Assay 2); select compounds that inhibit foot tapping with ID₅₀ ≤ 3mg/kg i.v., and preferably ID₅₀ ≤ 1mg/kg i.v. when administered immediately prior to central NK₁ agonist challenge, or ID₅₀ ≤ 30mg/kg p.o., and preferably ID₅₀ ≤ 10mg/kg p.o. 1 hour prior to challenge.
(iii) Determine central duration of action of compounds in gerbil foot tapping assay following intravenous administration 24 hours prior to central NK₁ agonist challenge; select compounds showing ≤ 25-fold loss of potency compared with ID₅₀ determined in step (ii) above with the proviso that ID₅₀ ≤ 10mg/kg i.v., and preferably ≤ 5mg/kg i.v. after 24 hour pre-treatment.
(iv) Determine oral bioavailability of compounds by pharmacokinetic analysis, activity in gerbil foot tapping assay following oral administration and/or by ability to inhibit cisplatin-induced emesis in ferrets (Assay 3); select compounds with ID₉₀ ≤ 3mg/kg p.o., and preferably ID₉₀ ≤ 1mg/kg p.o.

Particularly preferred compounds of use in the present invention are identified using steps (i) to (iv) followed by step (v):
(v) Determine activity of compounds in assays sensitive to conventional antipsychotic drugs (inhibition of distress vocalisations in guinea-pig pups (Assay 4)). Select compounds with ID₅₀ ≤ 20mg/kg, and preferably ID₅₀ ≤ 10mg/kg.

Yet further preferred compounds of use in the present invention may be selected from those compounds which satisfy the NK-1 receptor binding criteria of step (i) which, in addition, have ≤ 5-fold shift in affinity when incubated in the presence of human serum albumin (HSA) to show non-specific protein binding.

One example of a NK-1 receptor antagonist of use in the present invention is the compound 2-(R)-(1-(R)-(3,5-bis(trifluoroanethyl)phenyl)-ethoxy)-3-(S)-(4-fluorophenyl)-4-(3-(5-oxo-1H,4H-1,2,4-triazolo)methyl)-morpholine, the preparation of which is described in International Patent Specification No. WO 95/16679. In the aforementioned assays, this compound has the following activity:

| | |
|---|---|
| human NK-1 receptor binding | IC₅₀=0.1nM |
| gerbil foot-tapping (5 mins.) | ID₅₀=0.36mg/kg i.v. |
| gerbil foot-tapping (24 hrs.) | ID₅₀=0.33mg/kg i.v. |
| ferret emesis | ID₉₀<3mg/kg p.o. |
| guinea-pig vocalisation (4 hr. pre-treatment) | ID₅₀=0.73mg/kg p.o. |

The following example illustrates pharmaceutical compositions according to the present invention.

### EXAMPLE 1 Tablets containing 50-300mg of NK-1 antagonist

| | Amount mg | | |
|---|---|---|---|
| NK-1 antagonist | 50.0 | 100.0 | 300.0 |
| Microcrystalline cellulose | 80.0 | 80.0 | 80.0 |
| Modified food corn starch | 80.0 | 80.0 | 80.0 |
| Lactose | 189.5 | 139.5 | 139.5 |
| Magnesium Stearate | 0.5 | 0.5 | 0.5 |

The active ingredient, cellulose, lactose and a portion of the corn starch are mixed and granulated with 10% corn starch paste. The resulting granulation is sieved, dried and blended with the remainder of the corn starch and the magnesium stearate. The resulting granulation is then compressed into tablets containing 50mg, 100mg and 300mg of the NK-1 receptor antagonist per tablet.

## Claims

1. Use of an orally active, long acting, CNS-penetrant NK-1 receptor antagonist for the manufacture of a medicament adapted for oral administration for the treatment or prevention of a major depressive disorder selected from single or recurrent major depressive episodes, with or without psychotic features, catatonic features, melancholic features, atypical features or postpartum onset and, in the case of recurrent episodes, with or without interepisode recovery and with or without seasonal pattern, without concomitant therapy with other antidepressant agents,
wherein said NK-1 receptor antagonist is long acting as determined by its ability to inhibit foot-tapping in the gerbil with ≤ 25-fold loss of potency where the NK-1 receptor antagonist is administered 24 hours prior to NK-1 receptor agonist challenge compared with the potency when administered immediately prior to the NK-1 receptor agonist challenge, with the proviso that the ID₅₀≤10mg/kg i.v. after 24 hour pre-treatment.

2. A use according to claim 1 wherein the major depressive disorder encompasses a mood disorder selected from dysthymic disorder with early or late onset and with or without atypical features; dementia of the Alzheimer's type, with early or late onset, with depressed mood; mood disorders induced by alcohol, amphetamines, cocaine, hallucinogens, inhalants, opioids, phencylidine, sedatives, hypnotics, anxiolytics and other substances; schizoaffective disorder of the depressed type; and adjustment disorder with depressed mood.

3. A use according to claim 1 or claim 2 wherein the major depressive disorder results from a general medical condition.

4. A use as claimed in claim 3 wherein the general medical condition is selected from myocardial infarction, diabetes, miscarriage or abortion.

5. A use according to claim 1 wherein the treatment is in a patient who is non-responsive to heterocyclic antidepressants, SSRI's, mixed serotonin and norepinephrine selective reuptake inhibitors, dopamine reuptake inhibitors or MAOIs, or for whom heterocyclic antidepressants, SSRI's, mixed serotonin and norepinephrine selective reuptake inhibitors, dopamine reuptake inhibitors or MAOIs are contraindicated.

6. A use according to any one of claims 1 to 5 wherein the orally active, long acting CNS-penetrant NK-1 receptor antagonist is:
(2-(S)-(3, 5-bis(trifluoromethyl)benzyloxy)-3(S)-(4-fluorophenyl)-4-(3-(5-oxo-1H,4H-1,2,4-triazolo)methyl)morpholine;
2-(R)-(1-(R)-(3,5-bis(trifluoromethyl)phenyl)ethoxy)-4-(3-(5-oxo-1H,4H-1,2,4-triazolo)methyl)-3-(S)-phenyl-morpholine;
2-(S)-(3,5-bis(trifluoromethyl)benzyloxy)-4-(3-(5-oxo-1H,4H-1,2,4-triazolo)methyl)-3-(S)-phenyl-morpholine;
2-(R)-(1-(R)-(3,5-bis(trifluoromethyl)phenyl)ethoxy)-3-(S)-(4-fluorophenyl)-4-(3-(5-oxo-1H,4H-1,2,4-triazolo)methyl)morpholine;
2-(R)-(1-(R)-(3,5-bis(trifluoromethyl)phenyl)ethoxy)-4-(5-(N,N-dimethylamino)methyl-1,2,3-triazol-4-yl)methyl-3-(S)-phenylmorpholine;
2-(R)-(1-(R)-(3,5-bis(trifluoromethyl)phenyl)ethoxy)-4-(5-(N,N-dimethylamino)methyl-1,2,3-triazol-4-yl)methyl-3-(S)-(4-fluorophenyl)morpholine;
2-(R)-(1-(R)-(3,5-bis(trifluoromethyl)phenyl)ethoxy)-3-(S)-(4-fluorophenyl)-4-(3-(4-monophosphoryl-5-oxo-1H-1,2,4-triazolo)methyl)morpholine;
2-(R)-(1-(R)-(3,5-bis(trifluoromethyl)phenyl)ethoxy)-3-(S)-(4-fluorophenyl)-4-(3-(1-monophosphoryl-5-oxo-1H-1,2,4-triazolo)methyl)morpholine;
2-(R)-(1-(R)-(3,5-bis(trifluoromethyl)phenyl)ethoxy)-3-(S)-(4-fluorophenyl)-4-(3-(2-monophosphoryl-5-oxo-1H-1,2,4-triazolo)methyl)morpholine;
2-(R)-(1-(R)-(3,5-bis(trifluoromethyl)phenyl)ethoxy)-3-(S)-(4-fluorophenyl)-4-(3-(5-oxyphosphoryl-1H-1,2,4-triazolo)methyl)morpholine;
2-(S)-(1-(R)-(3,5-bis(trifluoromethyl)phenyl)ethoxy)-3-(S)-(4-fluorophenyl)-4-(3-(1-monophosphoryl-5-oxo-4H-1,2,4-triazolo)methyl)morpholine;
2-(R)-(1-(R)-(3,5-bis(trifluoromethyl)phenyl)ethoxy)-4-(4-N,N-dimethylaminobut-2-yn-yl)-3-(S)-(4-fluorophenyl)morpholine;
(3S,5R,6S)-3-[2-cyclopropoxy-5-(trifluoromethoxy)phenyl]-6-phenyl-1-oxa-7-aza-spiro[4.5]decane;
(3R,5R,6S)-3-[2-cyclopropoxy-5-(trifluoromethoxy)phenyl]-6-phenyl-1-oxa-7-aza-spiro[4.5]decane;
(±)-(2*R*3*R*,2*S*3*S*)-N-{[2-cyclopropoxy-5-(trifluoromethoxy)phenyl]methyl}-2-phenylpiperidin-3-amine;
or a pharmaceutically acceptable salt thereof.

## Patentansprüche

1. Die Verwendung eines oral wirksamen, langwirkenden, ZNS-penetrierenden NK-1-Rezeptorantagonisten zur Herstellung eines zur oralen Verabreichung hergerichteten Medikaments zur Behandlung oder Prävention einer schweren depressiven Störung, ausgewählt aus einzelnen oder rezidivierenden schweren depressiven Episoden mit oder ohne psychotische(n) Symptome(n), katatonische(n) Symptome(n), melancholische(n) Symptome(n), atypische(n) Symptome(n) oder Depression mit postpartalem Beginn, und, im Falle von rezidivierenden Episoden, mit oder ohne Genesung zwischen den Episoden und mit oder ohne saisonalem Muster, ohne begleitende Therapie mit anderen Antidepressiva,
wobei der NK-1-Rezeptorantagonist langwirkend ist, wie festgestellt durch sein Vermögen zur Inhibierung des Fußklopfens bei der Wüstenrennmaus mit einem ≤ 25fachen Wirksamkeitsverlust, wenn der NK-1-Rezeptorantagonist 24 Stunden vor der Herausforderung mit einem NK-1-Rezeptoragonisten verabreicht wird, im Vergleich zu der Wirksamkeit, wenn er unmittelbar vor der Herausforderung mit einem NK-1-Rezeptoragonisten verabreicht wird, mit der Maßgabe, daß der ID₅₀-Wert nach 24 Stunden Vorbehandlung ≤ 10 mg/kg i.v. beträgt.

2. Eine Verwendung gemäß Anspruch 1, wobei die schwere depressive Störung eine Gemütsstörung umfaßt, ausgewählt aus dysthymischer Störung mit frühem oder spätem Beginn und mit oder ohne atypische(n) Symptome(n); Demenz vom Alzheimer-Typ mit frühem oder spätem Beginn mit depressiver Stimmung; durch Alkohol, Amphetamine, Kokain, Halluzinogene, Inhalantien, Opioide, Phencyclidin, Sedativa, Hypnotika, Anxiolytika und andere Substanzen hervorgerufenen Gemütsstörungen; schizoaffektiver Störung vom depressiven Typ und Anpassungsstörung mit depressiver Reaktion.

3. Eine Verwendung gemäß Anspruch 1 oder Anspruch 2, wobei die schwere depressive Störung von einem allgemeinen medizinischen Zustand herrührt.

4. Eine wie in Anspruch 3 beanspruchte Verwendung, wobei der allgemeinen medizinische Zustand ausgewählt ist aus Myokardinfarkt, Diabetes, Fehlgeburt oder Abtreibung.

5. Eine Verwendung gemäß Anspruch 1, wobei die Behandlung bei einem Patienten erfolgt, der nicht auf heterocyclische Antidepressiva, SSRIs, gemischt Serotonin und Norepinephrin selektive Wiederaufnahmeinhibitoren, Dopaminwiederaufnahmeinhibitoren oder MAOIs anspricht oder für den heterocyclische Antidepressiva, SSRIs, gemischt Serotonin und Norepinephrin selektive Wiederaufnahmeinhibitoren, Dopaminwiederaufnahmeinhibitoren oder MAOIs kontraindiziert sind.

6. Eine Verwendung gemäß irgendeinem der Ansprüche 1 bis 5, wobei der oral wirksame, langwirkenden, ZNS-penetrierenden NK-1-Rezeptorantagonist ist:
(2-(S)-(3,5-Bis(trifluormethyl)benzyloxy)-3(S)-(4-fluorphenyl)-4-(3-(5-oxo-1H,4H-1,2,4-triazol)methyl)morpholin,
2-(R)-(1-(R)-(3,5-Bis(trifluormethyl)phenyl)ethoxy)-4-(3-(5-oxo-1H,4H-1,2,4-triazol)methyl)-3-(S)-phenylmorpholin,
2-(S)-(3,5-Bis(trifluormethyl)benzyloxy)-4-(3-(5-oxo-1H,4H-1,2,4-triazol)methyl)-3-(S)-phenylmorpholin,
2-(R)-(1-(R)-(3,5-Bis(trifluormethyl)phenyl)ethoxy)-3-(S)-(4-fluorphenyl)-4-(3-(5-oxo-1H,4H-1,2,4-triazol)methyl)morpholin,
2-(R)-(1-(R)-(3,5-Bis(trifluormethyl)phenyl)ethoxy)-4-(5-(N,N-dimethylamino)methyl-1,2,3-triazol-4-yl)methyl-3-(S)-phenylmorpholin,
2-(R)-(1-(R)-(3,5-Bis(trifluormethyl)phenyl)ethoxy)-4-(5-(N,N-dimethylamino)methyl-1,2,3-triazol-4-yl)methyl-3-(S)-(4-fluorphenyl)morpholin,
2-(R)-(1-(R)-(3,5-Bis(trifluormethyl)phenyl)ethoxy)-3-(S)-(4-fluorphenyl)-4-(3-(4-monophosphoryl-5-oxo-1H-1,2,4-triazol)-methyl)morpholin,
2-(R)-(1-(R)-(3,5-Bis(trifluormethyl)phenyl)ethoxy)-3-(S)-(4-fluorphenyl)-4-(3-(1-monophosphoryl-5-oxo-1H-1,2,4-triazol)-methyl)morpholin,
2-(R)-(1-(R)-(3,5-Bis(trifluormethyl)phenyl)ethoxy)-3-(S)-(4-fluorphenyl)-4-(3-(2-monophosphoryl-5-oxo-1H-1,2,4-triazol)-methyl)morpholin,
2-(R)-(1-(R)-(3,5-Bis(trifluormethyl)phenyl)ethoxy)-3-(S)-(4-fluorphenyl)-4-(3-(5-oxyphosphoryl-1H-1,2,4-triazol)methyl)morpholin,
2-(S)-1-(R)-(3,5-Bis(trifluormethyl)phenyl)ethoxy)-3-(S)-(4-fluorphenyl)-4-(3-(1-monophosphoryl-5-oxo-4H-1,2,4-triazol)methyl)-morpholin,
2-(R)-(1-(R)-(3,5-Bis(trifluormethyl)phenyl)ethoxy)-4-(4-N,N-dimethylaminobut-2-inyl)-3-(S)-(4-fluorphenyl)morpholin,
(3S,5R,6S)-3-[2-Cyclopropoxy-5-(trifluormethoxy)phenyl]-6-phenyl-1-oxa-7-azaspiro[4.5)decan,
(3R,5R,6S)-3-[2-Cyclopropoxy-5-(trifluormethoxy)phenyl]-6-phenyl-1-oxa-7-azaspiro[4.5]decan,
(±)-(2R3R,2S3S)-N-{[2-Cyclopropoxy-5-(trifluormethoxy)phenyl]-methyl}-2-phenylpiperidin-3-amin,
oder ein pharmazeutisch annehmbares Salz davon.

## Revendications

1. Utilisation d'un antagoniste au récepteur NK-1 pénétrant le SNC, à action longue, actif oralement, pour la fabrication d'un médicament adapté à l'administration orale pour le traitement ou la prévention d'un trouble dépressif majeur choisi parmi un seul épisode ou des épisodes dépressifs majeurs récurrents, avec ou sans caractères psychotiques, catatoniques, mélancoliques, atypiques ou attaque post-partum et, dans le cas des épisodes récurrents, avec ou sans inter-épisode de récupération et avec ou sans schéma saisonnier, sans thérapie concomitante avec d'autres agents antidépresseurs, dans laquelle ledit antagoniste au récepteur NK-1 est à action longue comme déterminé par sa capacité à inhiber le tapotement du pied chez la gerbille, avec une perte de ≤ 25 fois d'efficacité quand l'antagoniste au récepteur au NK-1 est administré 24 heures avant le rappel d'agoniste au récepteur NK-1, en comparaison de l'efficacité quand il est administré immédiatement avant le rappel d'agoniste au récepteur NK-1, pourvu que la ID₅₀ soit ≤ 10 mg/kg en i. v. après 24 heures de prétraitement.

2. Utilisation selon la revendication 1, dans laquelle le trouble dépressif majeur englobe un trouble de l'humeur choisi parmi le trouble dysthymique avec une attaque précoce ou tardive et avec ou sans caractères atypiques ; la démence du type d'Alzheimer, avec une attaque précoce ou tardive, avec une humeur dépressive ; les troubles de l'humeur induits par l'alcool, les amphétamines, la cocaïne, les hallucinogènes, les inhalants, les opiacés, la phencylidine, les sédatifs, les hypnotiques, les anxiolytiques et d'autres substances ; le trouble schizo-affectif du type déprimé ; et un trouble de l'adaptation avec une humeur déprimée.

3. Utilisation selon les revendications 1 ou 2, dans laquelle le trouble dépressif majeur provient d'une affection médicale générale.

4. Utilisation selon la revendication 3, dans laquelle l'affection médicale générale est choisie parmi l'infarctus du myocarde, le diabète, la fausse-couche ou l'avortement.

5. Utilisation selon la revendication 1, dans laquelle le traitement se fait chez un patient qui ne répond pas aux antidépresseurs hétérocycliques, aux SSRI, aux inhibiteurs de capture sélective de la sérotonine et de la noradrénaline mélangés, aux inhibiteurs de capture de la dopamine ou aux MAOI ou pour lesquels les antidépresseurs hétérocycliques, les SSRI, les inhibiteurs de capture sélective de la sérotonine et de la noradrénaline mélangés, les inhibiteurs de capture de la dopamine ou les MAOI sont contre-indiqués.

6. Utilisation selon l'une quelconque des revendications 1 à 5, dans laquelle l'antagoniste au récepteur NK-1 pénétrant le SNC, à action longue, actif oralement, est :
la (2(S)-(3,5-bis (trifluorométhyl) benzyloxy)-3(S)-(4-fluorophényl)-4-(3-(5-oxo-1H,4H-1,2,4-triazolo) méthyl) morpholine ;
la 2(R)-(1-(R)-3,5-bis (trifluorométhyl) phényl) éthoxy)-4-(3-(5-oxo-1H,4H-1,2,4-triazolo) méthyl)-3(S)-phénylmorpholine ;
la 2(S)-(3,5-bis (trifluorométhyl) benzyloxy)-4-(3-(5-oxo-1H,4H-1,2,4-triazolo) méthyl)-3(S)-phénylmorpholine ;
la 2(R)-(1-(R)-(3,5-bis (trifluorométhyl) phényl) éthoxy)-3(S)-(4-fluorophényl)-4-(3-(5-oxo-1H,4H-1,2,4-triazolo) méthyl) morpholine ;
la 2(R)-(1-(R)-(3,5-bis (trifluorométhyl) phényl) éthoxy)-4-(5-(N,N-diméthylamino) méthyl-1,2,3-triazol-4-yl) méthyl-3(S)-phénylmorpholine ;
la 2(R)-(1-(R)-(3,5-bis (trifluorométhyl) phényl) éthoxy)-4-(5-(N,N-diméthylamino) méthyl-1,2,3-triazol-4-yl) méthyl-3(S)-(4-fluorophényl) morpholine ;
la 2(R)-(1-(R)-(3,5-bis (trifluorométhyl) phényl) éthoxy)-3(S)-(4-fluorophényl)-4-(3-(4-monophosphoryl-5-oxo-1H-1,2,4-triazolo) méthyl) morpholine ;
la 2(R)-(1-(R)-(3,5-bis (trifluorométhyl) phényl) éthoxy)-3(S)-(4-fluorophényl)-4-(3-(1-monophosphoryl-5-oxo-1H-1,2,4-triazolo) méthyl) morpholine ;
la 2(R)-(1-(R)-(3,5-bis (trifluorométhyl) phényl) éthoxy)-3(S)-(4-fluorophényl)-4-(3-(2-monophosphoryl-5-oxo-1H-1,2,4-triazolo) méthyl) morpholine ;
la 2(R)-(1-(R)-(3,5-bis (trifluorométhyl) phényl) éthoxy)-3(S)-(4-fluorophényl)-4-(3-(5-oxyphosphoryl-lH-1,2,4-triazolo) méthyl) morpholine ;
la 2(S)-(1-(R)-(3,5-bis (trifluorométhyl) phényl) éthoxy)-3(S)-(4-fluorophényl)-4-(3-(1-monophosphoryl-5-oxo-4H-1,2,4-triazolo) méthyl) morpholine ;
la 2(R)-(1-(R)-(3,5-bis (trifluorométhyl) phényl) éthoxy)-4-(4-N,N-diméthylaminobut-2-ynyl)-3(S)-(4-fluorophényl) morpholine ;
le (3S,5R,6S)-3-[2-cyclopropoxy-5-(trifluorométhoxy) phényl)-6-phényl-1-oxa-7-azaspiro [4,5] décane ;
le (3R,5R,6S)-3-[2-cyclopropoxy-5-(trifluorométhoxy) phényl]-6-phényl-1-oxa-7-azaspiro [4,5] décane ;
la (±) (*2R3R,2S3S*)-N-{[2-cyclopropoxy-5-(trifluorométhoxy) phényl] méthyl}-2-phénylpipéridin-3-amine ;
ou un sel pharmaceutiquement acceptable de ceux-ci.
